# EUROPEAN PATENT SPECIFICATION

(11) **EP 1 850 885 B1**
(45) Date of publication and mention of the grant of the patent: **06.01.2010**
(21) Application number: 06701854.9
(22) Date of filing: 14.02.2006
(51) Int. Cl.: A61L 9/00, A61L 9/12

(54) **DEVICE FOR DISPENSING VOLATILE SUBSTANCES INTO THE AMBIENT ENVIRONMENT**
VORRICHTUNG ZUR ABGABE VON FLÜCHTIGEN SUBSTANZEN IN DIE UMGEBUNG
DISTRIBUTEUR DE SUBSTANCES VOLATILES DANS LE MILIEU AMBIANT

(30) Priority: 16.02.2005 EP 05290340
(43) Date of publication of application: 07.11.2007
(73) Proprietor: Givaudan SA, 1214 Vernier-Genève (CH)
(72) Inventor: BLONDEAU, Philippe, F-75019 Paris (FR); BRESSON BOIL, Alice, 95220 Herblay (FR); GEFFROY, Cédric, F-86000 Poitiers (FR)
(74) Representative: Givaudan Patents
(86) International application number: PCT/CH2006/000097
(87) International publication number: WO 2006/086904

(56) References cited:
- WO-A-2004/087225
- DE-A1- 4 430 291
- GB-A- 2 395 126
- US-A- 4 293 095
- US-A1- 2004 217 188
- PATENT ABSTRACTS OF JAPAN vol. 2003, no. 02, 5 February 2003 (2003-02-05) & JP 2002 291858 A (KOBAYASHI PHARMACEUT CO LTD), 8 October 2002 (2002-10-08)

## Description

The present invention relates to an apparatus for dispensing volatile substances. More particularly the present invention relates to a wick-based dispensing device for the delivery of volatile substances from a bi-layer liquid to an ambient environment by evaporation.

Wick-based dispensing devices for the dispensing into the ambient environment of volatile liquids are well known in the art. One very common type of such dispensing devices consists essentially of a reservoir containing the volatile liquid and a wick immersed in the liquid and communicating with a porous substrate optionally having a broad evaporation surface. Such dispensing devices employ capillary phenomenon to provide the motive dispensing force. The wick operates to transport the liquid from the interior of the reservoir to the ambient environment, into which it evaporates. Such devices may additionally comprise auxiliary means, such as heating elements and/or fans. The liquids used in such systems consist typically of active, volatile substances, and a solvent in the case of heated devices and non-heated, fan-driven devices, or consisting of essentially water, solvent, solubilizer and active, volatile substances, when used in non-heated, non-fan-driven devices.

One problem with such systems is that, due to the formulation of the aqueous-based liquid, the wicking material tends to clog, rendering the device less effective. Moreover, no clear end of life signal (EOL) may be observed in these systems as in general a viscous residue stays in the reservoir. Furthermore, the presence of solvent, in particular in larger amounts, is costly and not environmentally friendly.

Certain attempts have recently been made to overcome these problems by using solvent and solubilizer-free aqueous-based liquid compositions, resulting in bi-layer formulations, in conventional wick-based dispensing devices. The problem with these devices is that they do not allow a concomitant evaporation of both layers.

It has now been found that it is possible to provide a device that at least substantially overcomes the problems of the art. The invention therefore provides a dispensing device adapted to release a volatile liquid into the ambient environment, the device comprising a reservoir containing a volatile liquid in the form of two layers, from which reservoir extend a first and a second capillary element, each element having a lower portion immersed in both layers of the liquid and an upper portion extending into the ambient environment, the first capillary element being porous with respect to the liquid forming the upper layer only and the second capillary element being porous with respect to the liquid of both layers and the second capillary element having on its surface a barrier layer that is impermeable to the liquid forming the upper layer.

The invention further provides a method of dispensing into an ambient environment a volatile liquid that is in the form of two layers by transporting the liquid from the reservoir to the environment by means of a first and second capillary element, each element having a lower portion immersed in both layers of the liquid and an upper portion extending into the ambient environment, the first capillary element being porous with respect to the liquid forming the upper layer only and the second capillary element being porous with respect to the liquid of both layers and the second capillary element having on its surface a barrier layer that is impermeable to the liquid forming the upper layer.

The reservoir may be made of any material that is commonly used for air-freshener devices, e.g. glass or plastic. Such materials are preferred, which are stable in the presence of fragrance materials, e.g. essential oils. From this reservoir extend the first and second capillary elements. These may be located in place by any convenient means for example, their fitting tightly within a liquid-tight plug, which blocks an orifice in the reservoir, through which the capillary elements pass. Such a plug preferably comprises a vent, which permits equalisation of pressure. Alternatively, any means for equalisation may be used, such as vented wicks as described in WO 03092750, which is incorporated herein by reference.

The barrier layer making the second capillary element impermeable to the liquid of the upper layer may take any suitable form, for example, a liquid coating applied thereto and allowed harden or dry, or a sleeve in the form of an adhesive tape or a tight-fitting tubular member of any suitable material placed thereon. The tubular member may be made, for example, of polyethylene, polypropylene, PETG (polyethylene glycol terephatalate), Barex^{®}resins from BP Chemicals Ltd., polyester or polystyrene. The barrier layer may be such that it is impermeable to the liquid of both layers and is extended along the second capillary element to such an extent that only the liquid forming the lower layer is absorbed, that is, the barrier layer may extend only along that part of the second capillary element that passes through the liquid forming the upper layer and a sufficient distance into the liquid forming the lower layer, to allow for the lowering by evaporation of the boundary of the two layers.

In a preferred embodiment of the invention, the major part of the lateral surface of the lower portion of the second capillary element is covered by the barrier material and essentially only the cross-section of the second capillary element is in contact with the liquid having no barrier layer. By "major part" is meant that, at full liquid load, at least half of the capillary element in contact with the liquid of the lower layer, preferably 3/4 of the capillary element, more preferably the complete lateral surface is covered by the barrier material. The proportions given refer to a filled device not yet in use to release a volatile liquid by evaporation.

In a further embodiment of the invention, the first capillary element may be arranged in such a way that it substantially covers the outer surface of the barrier layer of the second capillary element. The barrier layer may be such that it is extended along the second capillary element to such an extent that only the liquid forming the lower layer is absorbed, that is, the major part of the lateral surface of the upper and lower part of the second capillary element is covered by the barrier layer, essentially only the cross-sections having no barrier.

The volatile liquid forming two layers consists of an aqueous phase forming the lower layer and a hydrophobic phase forming the upper layer. The hydrophobic phase consists essentially of fragrance. By "fragrance" is meant a single fragrance material or a mixture of fragrance materials selected from such classes as acids, esters, alcohols, aldehydes, ketones, lactones, nitriles, ethers, acetates, hydrocarbons, sulfur- nitrogen- and oxygen-containing heterocyclic, polycyclic and macrocyclic compounds, as well essential oils of natural or synthetic origin. Such fragrance materials are described, for example, in S. Arctander, Perfume Flavors and Chemicals Vols. 1 and 2, Arctander, Montclair, NJ USA 1969. The fragrance optionally may comprise odourless liquids such as benzyl benzoate, isopropylmyristate, and hydrocarbon derivatives, for example Isopar^{®} from Exxon. The appropriate choice of the liquid not only depends on the amount in which it may be used but also on the nature of the fragrance as will be fully appreciated by the person skilled in the art. Preferably, the fragrance comprises not more than 25% by weight, preferably not more than 20% by weight of these liquids based on the total amount of the hydrophobic phase.

By "aqueous phase" is meant a hydrophilic liquid comprising at least 40% by weight, preferably 50 to 100% by weight of water, based on the total amount of the aqueous phase.

The ratio of the aqueous phase and the hydrophobic phase depends on the particular capillary elements chosen, and is chosen preferably in such a way that both phases are almost completely evaporated at the same time. The exact ratio can be easily determined by a person skilled in the art.

The first capillary element, being porous with respect to the liquid forming the upper layer, may be made of any material suitable for the transfer of the liquid forming the upper layer by capillary action from the lower portion to the upper portion of the capillary element. Examples of suitable materials include polyolefins, e.g. PE (polyethylene) and PP (polypropylene) or a combination thereof.

The second capillary element, being porous with respect to the liquid of both layers, i.e. the aqueous and the hydrophobic layer, may be made of any material suitable for the transfer by capillary action of the liquid of both layers. Examples of suitable materials include polyester, e.g. PET (polyethylenterephthalate), cellulose and cellulose acetate, and combinations thereof. Preferably the second capillary element is made of cellulose. The second capillary element may also be made of polyolefins, e.g. PE (polyethylene) and PP (polypropylene) or a combination thereof, which is further treated to make the material suitable to transfer by capillary action the liquid of both layers. This can be achieved, for example, by dipping the second capillary element made of pololefins into an aqueous solution comprising an anionic surfactant such as sodium ether sulfate, or a nonionic surfactant such as ethoxylated fatty alcohol, preferably at about 20 weight % based on the aqueous solution, resulting in an impregnated material, which is allowed to dry, preferably over night at room temperature, before use.

The shape of the capillary elements is not critical. They can be, for example, of square, rectangular, circular or annular section. The lengths of the capillary elements depend primarily on the height of the reservoir. They are chosen preferably in such a way that they reach the bottom of the reservoir and thus the entire contents of the reservoir can be evaporated.

In a further and preferred embodiment of the invention, the liquid forming the upper and/or the liquid forming the lower layer comprise(s) a dye, preferably of a distinctive color. Once the volatile liquid reaches the end point of evaporation, the first and second capillary elements have the same color, i.e. the color of the liquid forming the upper layer. This visual signal indicates to the consumers that the product is finished and must be replaced.

Suitable dyes for the hydrophobic phase may be selected from Puricolor^{®} blue FBL5, Puricolor^{®} blue ABL9, Puricolor^{®} green U3 and Puricolor^{®} yellow AYE23 from Ciba and suitable dyes for the aqueous phase may be selected from Covasol green W7039 from Wackherr and Vitasyn^{®} orange RGL 90 from Clariant.

A device according to the present invention is particularly suitable for wick-based dispensing devices further comprising a heating element. In addition the wick-based dispensing device may have a fan.

The invention will now further be described with reference to the drawings, which depict a preferred embodiment of the invention and which are not to be construed as limiting in any way.
Fig. 1 is a vertical cross-section through an embodiment of the invention with a full charge of volatile liquid.
Fig. 2 is a vertical cross-section through the embodiment of Figure 1 after evaporation of the major part of the volatile liquid.
Fig. 3A is a vertical cross-section through a further embodiment of the present invention.
Fig. 3B depicts a cross-section view of the embodiment of Fig. 3A, taken along line CC'.

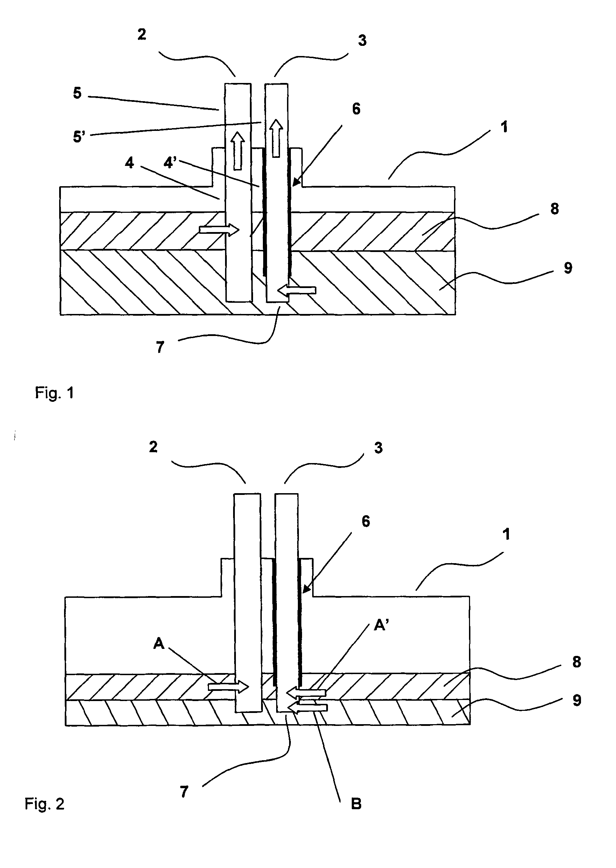

Referring to Fig.1, a wick-based dispensing device comprises a reservoir (1), which contains a volatile liquid in form of an upper hydrophobic layer comprising fragrance (8) and an aqueous lower layer (9), these layers comprising different coloured dyes. Extending from the liquid into an atmosphere through an opening in the reservoir are two capillary elements in the form of wicks (2) and (3), the wicks having upper portions, (5) and (5') respectively, exposed to the atmosphere and lower proportions, (4) and (4') respectively, exposed to the liquid. The materials of the wicks are selected such that the wick (2) can absorb and convey to the atmosphere only the liquid of layer (8), whereas the material of the wick (3) is capable of absorbing and conveying the liquid of both layers.

The major part of the lower portion (4') of the wick (3) is covered by a tight-fitting polyethylene sleeve (6), making wick (3) impermeable to the liquid of the upper layer (8). At full liquid load, the sleeve (6) extends below the boundary between the layers, thus ensuring that only a lower end (7) of the lower portion (4') of wick (3) is exposed only to liquid layer (9) and that only this liquid can evaporate through it until most of the volatile liquid is evaporated.

In operation, initially as shown in Figure 1, upper liquid layer (8) is conveyed through and evaporates from wick (2) and lower liquid layer (9) is conveyed through the lower end (7) and evaporates from the upper portion (5') of wick (3), as indicated by the white arrows in that drawing.

As shown in Figure 2, this remains the case until the boundary between the layers falls below the lower end of the sleeve (6) of wick (3). At this point, both liquid layers (8) and (9) are conveyed by wick (3). This is depicted by white arrows A' and B, whereas wick (2) continues to convey only upper liquid layer (8), as depicted by white arrow A. As a result of exposure of the lower end (7) of wick (3) to the upper liquid layer (8) and the consequent conveyance of that liquid through the wick (3), there is a noticeable change of colour of wick (3). This indicates that the device has reached the end of its life and that it should be replaced

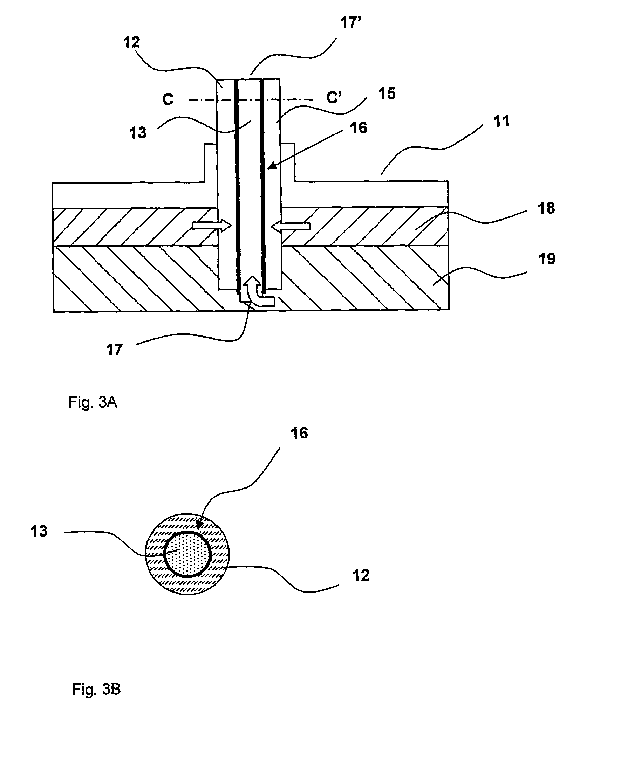

Referring to Fig. 3A, a wick-based dispensing device is shown following the same working principle as describe in Fig. 1 and 2. The only difference is that, instead of two individual wicks, one single wick system as also shown in further details in Fig. 3B is used. The wick-based dispensing device comprises a reservoir (11) containing a volatile liquid in form of a lower (19) and an upper liquid layer (18). Extending form the liquid into an atmosphere through an opening in the reservoir is a wick, assembled from a second capillary element (13) having on its lateral surface a tight-fitting sleeve (16) which is covered by a first capillary element (12). The material of the wick is selected such that the first capillary element (12) can absorb and convey to the atmosphere only the liquid of layer (18), whereas the material of the inner capillary element (13) is capable of absorbing and conveying both liquid layers. The outer layer (12) of the wick is a tube through which the second capillary element in form of a rode (13) made of cellulose is guided. The cellulose rod is covered by a tight-fitting sleeve (16) making it impermeable to the liquid (18) of the upper layer.

In operation, initially shown in Fig. 3A, upper liquid layer (18) is conveyed through and evaporates from the upper portion (15) of the outer layer (12) of the wick and lower liquid layer (19) is conveyed through the lower end (17) and evaporates from the upper end (17') of the inner part (13) of the wick, as indicated by the white arrows in Fig. 3A.

The invention has been described in terms of dispensing fragrance. However, the device according to the invention may deliver other ingredients such as malodour counteracting ingredients, insect repellents, or materials having insecticidal activities, substance having antimicrobial activities, or mixtures thereof. These other ingredients may be present in both, the aqueous and/or hydrophilic phase. In addition to these active principals, fragrance compositions may comprise excipients such as dyes and UV-absorbers.

In a further embodiment of the present invention the volatile liquid forming the two layers consist of an aqueous phase comprising up to 60% by weight, preferably from 1 to 50 % by weight, of ingredient(s) selected from malodour counteracting ingredients insect repellents, water soluble liquids, materials having insecticidal activities, substance having antimicrobial activities, and mixtures thereof, based on the total amount of the aqueous phase.

## Claims

1. A device adapted to release a volatile liquid into the ambient environment, the device comprising a reservoir (1; 11) containing a volatile liquid in the form of two layers (8, 9; 18, 19), from which reservoir extend a first and a second capillary element (2, 3; 12, 13), each element having a lower portion (4, 4') immersed in both layers of the liquid and an upper portion (5, 5'; 15) extending into the ambient environment, the first capillary element (2; 12) being porous with respect to the liquid forming the upper layer (8; 18) only and the second capillary element (3; 13) being porous with respect to the liquid of both layers and the second capillary element having on its surface a barrier layer (6; 16) that is impermeable to the liquid forming the upper layer.

2. A device according to claim 1 wherein the second capillary element (3; 13) is made of a material selected from polyester, cellulose and cellulose acetate, or a combination thereof.

3. A device according to claim 1 wherein the second capillary element (3; 13) is made of polyolefin, which is further treated to make the material suitable to transfer by capillary action the liquid of both layers (8, 9, 18, 19).

4. A device according to any one of the preceding claims wherein the first capillary element (2; 12) is made of polyolefin.

5. A device according to any one of the preceding claims wherein the first capillary element (2; 12) is arranged in such a way that it substantially covers the outer surface of the barrier layer (16) of the second capillary element.

6. A device according to claim 1 wherein at least the upper layer (8; 18) of the volatile liquid comprises a dye, which once an end point of evaporation of the volatile liquid is reached results in coloration of the first and second capillary element (2, 3; 12, 13) such that both capillary elements having the same color.

7. A method of dispensing into an ambient environment a volatile liquid that is in the form of two layers (8, 9, 18, 19) by transporting the liquid from a reservoir (1, 11) to the environment by means of a first and second capillary element (2, 3 ; 12, 13), each element having a lower portion (4, 4') immersed in both layers of the liquid and an upper portion (5, 5'; 15) extending into the ambient environment, the first capillary element (2, 12) being porous with respect to the liquid forming the upper layer (8, 18) only and the second capillary element (3, 13) being porous with respect to the liquid of both layers and the second capillary element having on its surface a barrier layer (6, 16) that is impermeable to the liquid forming the upper layer.

## Patentansprüche

1. Vorrichtung zur Freisetzung einer flüchtigen Flüssigkeit in die Umgebung, wobei die Vorrichtung ein Reservoir (1; 11) umfasst, das eine flüchtige Flüssigkeit in der Form von zwei Schichten (8, 9; 18, 19) enthält und aus dem sich ein erstes und ein zweites Kapillarelement (2, 3; 12, 13) erstrecken, die jeweils einen unteren Abschnitt (4, 4'), der in beiden Schichten der Flüssigkeit eingetaucht ist, und einen oberen Abschnitt (5, 5'; 15) haben, der sich in die Umgebung erstreckt, wobei das erste Kapillarelement (2; 12) nur bezüglich der die obere Schicht (8; 18) bildenden Flüssigkeit porös ist und das zweite Kapillarelement (3; 13) bezüglich der Flüssigkeit beider Schichten porös ist und auf seiner Oberfläche eine Sperrschicht (6; 16) hat, die für die die obere Schicht bildende Flüssigkeit undurchlässig ist.

2. Vorrichtung nach Anspruch 1, wobei das zweite Kapillarelement (3; 13) aus einem aus Polyester, Zellulose und Zelluloseacetat oder einer Kombination davon ausgewählten Material hergestellt ist.

3. Vorrichtung nach Anspruch 1, wobei das zweite Kapillarelement (3; 13) aus Polyolefin hergestellt ist, das weiterbehandelt ist, um das Material zum Transfer der Flüssigkeit beider Schichten (8, 9; 18, 19) durch Kapillarwirkung geeignet zu machen.

4. Vorrichtung nach einem der vorhergehenden Ansprüche, wobei das erste Kapillarelement (2; 12) aus Polyolefin hergestellt ist.

5. Vorrichtung nach einem der vorhergehenden Ansprüche, wobei das erste Kapillarelement (2; 12) so angeordnet ist, dass es die Außenfläche der Sperrschicht (16) des zweiten Kapillarelements im Wesentlichen abdeckt.

6. Vorrichtung nach Anspruch 1, wobei mindestens die obere Schicht (8; 18) der flüchtigen Flüssigkeit einen Farbstoff umfasst, der zur Färbung des ersten und des zweiten Kapillarelements (2; 3; 12; 13) führt, wenn die Verdampfung der flüchtigen Flüssigkeit einen Endpunkt erreicht hat, so dass beide Kapillarelemente dieselbe Farbe haben.

7. Verfahren zur Abgabe einer flüchtigen Flüssigkeit in der Form von zwei Schichten (8, 9; 18, 19) an die Umgebung, indem die Flüssigkeit mittels eines ersten und eines zweiten Kapillarelements (2, 3; 12, 13) aus einem Reservoir (1; 11) in die Umgebung transportiert wird, wobei jedes Element einen unteren Abschnitt (4, 4'), der in beiden Schichten der Flüssigkeit eingetaucht ist, und einen oberen Abschnitt (5, 5'; 15) hat, der sich in die Umgebung erstreckt, wobei das erste Kapillarelement (2; 12) nur bezüglich der die obere Schicht (8, 18) bildenden Flüssigkeit porös ist und das zweite Kapillarelement (3; 13) bezüglich der Flüssigkeit beider Schichten porös ist und auf seiner Oberfläche eine Sperrschicht (6; 16) hat, die für die die obere Schicht bildende Flüssigkeit undurchlässig ist.

## Revendications

1. Dispositif prévu pour libérer un liquide volatil dans l'environnement ambiant, le dispositif comprenant un réservoir (1 ; 11) contenant un liquide volatil sous forme de deux couches (8, 9 ; 18, 19), duquel réservoir s'étendent un premier et un deuxième élément capillaire (2, 3 ; 12, 13), chaque élément ayant une portion inférieure (4, 4') immergée dans les deux couches du liquide et une portion supérieure (5, 5' ; 15) s'étendant dans l'environnement ambiant, le premier élément capillaire (2 ; 12) étant poreux par rapport au liquide formant la couche supérieure (8 ; 18) uniquement et le deuxième élément capillaire (3 ; 13) étant poreux par rapport au liquide des deux couches et le deuxième élément capillaire ayant sur sa surface une couche barrière (6 ; 16) qui est imperméable au liquide formant la couche supérieure.

2. Dispositif selon la revendication 1, dans lequel le deuxième élément capillaire (3 ; 13) est fabriqué en un matériau choisi parmi le polyester, la cellulose et l'acétate de cellulose, ou une combinaison de ceux-ci.

3. Dispositif selon la revendication 1, dans lequel le deuxième élément capillaire (3 ; 13) est fabriqué en polyoléfine, qui est davantage traitée pour rendre le matériau approprié pour un transfert par action capillaire du liquide des deux couches (8, 9 ; 18, 19).

4. Dispositif selon l'une quelconque des revendications précédentes, dans lequel le premier élément capillaire (2 ; 12) est fabriqué en polyoléfine.

5. Dispositif selon l'une quelconque des revendications précédentes, dans lequel le premier élément capillaire (2 ; 12) est agencé de telle sorte qu'il couvre substantiellement la surface extérieure de la couche barrière (16) du deuxième élément capillaire.

6. Dispositif selon la revendication 1, dans lequel au moins la couche supérieure (8 ; 18) du liquide volatil comprend une teinture, qui, une fois qu'un point final d'évaporation du liquide volatil est atteint, entraine la coloration du premier et du deuxième élément capillaire (2 ; 3 ; 12 ; 13) de telle sorte que les deux éléments capillaires aient la même couleur.

7. Procédé de distribution dans un environnement ambiant d'un liquide volatil sous forme de deux couches (8, 9 ; 18, 19) en transportant le liquide d'un réservoir (1 ; 11) à l'environnement au moyen d'un premier et d'un deuxième élément capillaire (2, 3 ; 12, 13), chaque élément ayant une portion inférieure (4, 4') immergée dans les deux couches du liquide et une portion supérieure (5, 5' ; 15) s'étendant dans l'environnement ambiant, le premier élément capillaire (2 ; 12) étant poreux par rapport au liquide formant la couche supérieure (8, 18) uniquement et le deuxième élément capillaire (3 ; 13) étant poreux par rapport au liquide des deux couches, et le deuxième élément capillaire ayant sur sa surface une couche barrière (6 ; 16) qui est imperméable au liquide formant la couche supérieure.
